# EUROPEAN PATENT APPLICATION

(11) **EP 2 987 503 A1**
(43) Date of publication of application: **24.02.2016**
(21) Application number: 14382324.3
(22) Date of filing: 22.08.2014
(51) Int. Cl.: A61K 45/06, A61K 38/14, A61K 31/7016, A61K 31/7028, A61P 31/04

(54) **Methods and reagents for prevention and/or treatment of infection**

(71) Applicant: Institut d'Investigació Biomèdica de Bellvitge (IDIBELL), 08908 l'Hospitalet de Llobregat (ES)
(72) Inventor: Manez Mendiluce, Rafael, E-08908 Hospitalet de Llobregat (ES); Costa Vallés, Cristina, E-08908 Hospitalet de Llobregat (ES); Pérez Cruz, Magdiel, E-08908 Hospitalet de Llobregat (ES); Bello Gil, Daniel, E-08908 Hospitalet de Llobregat (ES)
(74) Representative: ABG Patentes, S.L.

(57) **Abstract**

The present invention relates to the field of therapeutics and, more in particular, to agents and compositions for the prevention and/or treatment of infection caused by bacteria of the gastrointestinal tract in a subject, said agents and compositions being based on agents comprising a terminal α-galactosyl moiety.

## Description

### TECHNICAL FIELD

The present invention relates to the field of therapeutics and, more in particular, to agents and compositions for the prevention and/or treatment of infections caused by bacteria of the gastrointestinal tract in a subject, said agents and compositions being based on agents comprising a terminal α-galactosyl moiety.

### BACKGROUND ART

Severe infections including sepsis (acute organ dysfunction secondary to infection) and septic shock (severe sepsis plus hypotension not reversed with fluid resuscitation) are major healthcare problems, affecting millions of individuals around the world each year, killing one in four (and often more) patients, and increasing in incidence. In the particular case of sepsis and septic shock, there is no specific treatment. Dotrecogin alpha (Xigris®) was considered for several years the unique therapy for these conditions. However, it has been recently retired from the market because a repeated clinical trial failed to show the efficacy observed in the initial PROWESS trial.

Two types of severe infections can be considered. Firstly, infection caused by exogenous microorganisms that colonize the upper airway including pneumococcus and meningococcus, wherein specific vaccines are available in order to prevent the disease related to the infection. The impact of vaccination in meningococcal infections is unquestionable, since the number of cases of meningococcal sepsis or meningitis in young adults during the last 25 years has dropped dramatically. The benefits of vaccination in relation to those cases of pneumococcal sepsis or meningitis are more controversial, since they still occur even in vaccinated subjects.

The second major group of severe infections is caused by endogenous enteric bacteria. The "gut origin of infections" hypothesis proposes that bacteria, which are normally resident within the lumen of the intestinal tract, translocate across the intestinal epithelial barrier and act as a source of infection at distant sites. A number of factors have been shown to predispose to bacterial translocation. These include shock with reduced splanchnic blood flow, parenteral nutrition, intestinal epithelial damage, and antibiotic therapy.

The clinical significance of bacterial translocation is noteworthy in nosocomial and neutropenic patient infections, playing an important role in the causation of sepsis. This may affect up to 12% of patients admitted to ICU (ICU-acquired sepsis) and 1.6% of people with cancer per year. Compared with the overall population, cancer patients are four times more likely to be hospitalized with severe infections, with in-hospital mortality of 37.8%. Other patients at risk for enteric bacterial infections are those submitted to surgery or to medical procedures. After cardiac surgery, 1.4% of patients develop serious infections with a mortality rate of 32%. Also, a recent study showed that, after general surgery, sepsis and septic shock occurred in 2.3% and 1.6% of patients, respectively, whilst the incidence of pulmonary embolism and heart attack were 0.3% and 0.2%, respectively. Death rates within 30 days were 5.4% for severe infection, 33.7% for septic shock, 9.1% for pulmonary embolism and 32% for heart attack. These data suggest that severe infections are a common and serious complication in those patients undergoing general surgery, and that they occur more frequently than pulmonary embolism or heart attack.

Some strategies have been proposed in the prevention and/or the treatment of infections caused by enteric bacteria in a subject. US8361441 B2 discloses a method for the prevention and/or the treatment of bacterial infection caused by Enterobacterioceae in a mammal that comprises the administration of a bacterial outer membrane protein A (OmpA) or its derivative. WO2004078209 A1 discloses a method for the treatment of an enteric disease caused by a Gram-negative bacteria, that includes administering a vaccine or a hyperimmune material raised against said vaccine to an individual, wherein the vaccine comprises one or more cell wall antigens reactive in a manner characteristic of O group serotypes, or reactive in a manner characteristic of lipopolysaccharide associated antigens, and at least some of said antigens are separated from bacterial cell walls or wall fragments.

However, there is still a need in the state of the art to identify suitable, effective agents for use in the treatment or the prevention of infections in a subject caused by bacteria of the gastrointestinal tract including enteric bacteria.

### BRIEF SUMMARY OF THE INVENTION

The authors of the present invention have found that the removal of antibodies against Galactose α1,3 Galactose residues mediated by GAS914 in a subject increases serum bactericidal activity against *Escherichia coli* blood isolates, proposing a role for those compounds comprising a terminal α-galactosyl moiety in the prevention and/or treatment of infections caused by bacteria of the gastrointestinal tract bacteria, particularly enteric bacteria, in a subject.

Thus, in a first aspect, the present invention is related to an agent comprising a terminal α-galactosyl moiety, for use in the prevention and/or treatment of an infection in a subject, wherein said infection is caused by bacteria of the gastrointestinal tract.

In a further aspect, the invention relates to a composition comprising:
(i) an agent comprising a terminal α-galactosyl moiety, and
(ii) an antibiotic.

In a further aspect, the invention relates to a composition as above for use in medicine.

In a last aspect, the invention relates to a composition as above for use in the prevention and/or treatment of infection caused by bacteria of the gastrointestinal tract in a subject.

### BRIEF DESCRIPTION OF THE FIGURES

**Figure 1** shows the effects of the removal of anti-Gal antibodies in Gal-KO mice. Survival rate **(A)** and welfare **(B)** in GAS914-treated mice (n = 17) before CLP and control mice (n = 17). **(C)** Survival rate in GAS914-treated mice 12h after CLP and control mice. **(D)** Anti-Gal IgM and IgG levels in GAS914-treated mice before CLP and control mice.
**Figure 2** shows an increase in IgG antibodies binding to *E. coli* in Gal-KO mice undergoing sepsis after removal of anti-Gal antibodies by GAS914.
**Figure 3** shows the bactericidal effect of GAS914 in Gal-KO mice, wherein serum reactivity in Gal-KO mice treated with GAS914 is similar to that observed in wild-type mice lacking anti-Gal antibodies. CLP: colon ligation and puncture.

### DETAILED DESCRIPTION OF THE INVENTION

The authors of the present invention have found that the removal of antibodies for galactose α1-3 galactose (anti-Gal antibodies) using a compound containing a terminal α-galactosyl moiety, in particular GAS914, increased serum IgG reactivity and bactericidal activity to blood bacterial isolates, in particular *Escherichia coli* isolates, in an animal model that generates natural anti-Gal antibodies (Gal-KO mice), said increase being similar to those levels observed in wild-type mice lacking anti-Gal antibodies (see Example 2). Based on this experimental data, the authors concluded that removal of anti-Gal antibodies increased serum bactericidal activity, and proposed a role for those compounds comprising a terminal α-galactosyl moiety in the prevention and/or treatment of infections in a subject caused by bacteria of the gastrointestinal tract bacteria, particularly enteric bacteria.

### Definitions

The term "α-galactosyl" or "α-galactosyl moiety" or "α-galactosyl residue", as used herein, relates to a terminal galactose residue in a molecule, i.e., to a glycosyl radical derived from α-galactose. In some contexts, the term alfa-galactosyl is related in particular to a unit of alfa-galactose bound to a second monosaccharide.

The term "agent comprising a terminal α-galactosyl moiety", as used herein, also known as "alpha-galactosyl agent", or "anti-Gal binding agent" refers to any molecule, or part of a molecule, with a terminal structure comprising Galα1-3Galβ1-4GlcNAc-R, Galα1-3Galβ1-3GlcNAc-R, or any carbohydrate chain with terminal Galα1-3Gal at the non-reducing end, or any molecule with terminal α-galactosyl unit, capable of binding the anti-Gal antibody, wherein said α-galactosyl unit may be bound to a second monosaccharide, such as galactose or glucose. The α-Gal epitope (also known as "alpha-galactosyl epitope", or "anti-Gal binding epitope") is synthesized by the glycosylation enzyme α-1,3-galactosyltransferase (α1,3GT) and it is expressed in very large amounts on the cells of non-primate mammals, prosimians and in New World monkeys. The α1,3GT gene was inactivated in ancestral Old World primates. Thus humans, apes, and Old World monkeys lack α-gal epitopes and produce high titer anti-Gal antibodies. In particular, the agent of the invention comprises a terminal α-galactosyl, wherein said α-galactosyl is selected from the group comprising terminal Galα1-3Gal, terminal Galα1-2Gal, terminal Galα1-6Gal, terminal Galα1-6Glc, and terminal α-galactose sugar unit(s) capable of binding anti-Gal antibodies. In a particular embodiment, the agent comprising a terminal α-galactosyl moiety is part of a glycolipid, a glycopeptide, a glycoprotein, a glycoconjugate or a liposome. The term "α-Gal glycolipid", refers to any glycolipid that has at least one α-gal moiety on its nonreducing end of the carbohydrate chain. The term "α-Gal liposomes" refers to any liposomes that have an α-gal moiety and are capable of binding the anti-Gal antibody.

The term "anti-Gal antibody", or "anti-Gal", as used herein, relates to an antibody that interacts specifically with the α-Gal epitope (Galα1-3Galβ1-4GlcNAc-R or Galα1-3Galβ1-3GlcNAc-R) on glycolipids, glycoproteins or other molecules comprising the alpha-galactosyl epitope. This antibody constitutes approximately 1% of circulating IgG in human serum and is produced, upon stimulation, by 1% of circulating B lymphocytes. Anti-Gal antibody is also present as IgA antibodies in body secretions such as saliva, milk and colostrum. It has been proposed that the antigenic source for the constant production of anti-Gal antibodies are the alpha-galactosyl-like epitopes found on many bacteria of the gastrointestinal flora. Whereas anti-Gal is abundant in humans, apes and Old World monkeys, it is absent from New World monkeys, prosimians and nonprimate mammals. The latter group of species produces, however, large amounts of alpha-galactosyl epitopes. It is estimated that anti-Gal appeared in ancestral Old World primates less than 28 million years ago, possibly as a result of an evolutionary event which exerted a selective pressure for the suppression of alpha-galactosyl epitopes expression by inactivation of the gene for the enzyme alpha 1,3 galactosyltransferase. This also resulted in the loss of immune tolerance to the alpha-galactosyl epitope and the production of anti-Gal. Anti-Gal antibodies bind *in vivo* to α-gal epitopes when administered to humans or Old World monkeys. This is particularly evident in the context of xenotransplantation, where *the in vivo* binding of anti-Gal to α-gal epitopes on transplanted pig heart or kidney is the main cause for the rapid rejection of such grafts in humans and Old World monkeys. Anti-Gal antibodies are responsible for the hyper-acute rejection of a xenograft.

The term "antibiotic", as used herein, relates to a chemical substance produced by a living being or a synthetic derivative thereof which at low concentrations kills or prevents the growth of certain classes of sensitive microorganisms, generally bacteria, although some antibiotics are also used for the treatment of infections by fungi or protozoa. Antibiotics are used in human, animal or horticultural medicine to treat infections caused by microorganisms. Antibiotics included in the present invention are, without limitation, aminoglycoside antibiotics, ansamycins, carbacefem, carbapenems, cephalosporins, glycopeptides, macrolides, monobactams, penicillins, polypeptides, quinolones, sulfonamides, tetracyclines and others such as arsphenamine, chloramphenicol, clindamycin, lincomycin, ethambutol, fosfomycin, fusidic acid, furazolidone, isoniazid, linezolid, metronidazole, mupirocin, nitrofurantoin, platensimycin, pyrazinamide, quinupristin/dalfopristin, rifampin or rifampicin, tinidazole, viomycin and capreomycin; preferably cephalosporins, tetracyclines, glycopeptides, carbapenems, polypeptides, rifampicin, aminoglycosides, sulfonamides, viomycin and capreomycin. In a preferred embodiment the antibiotic is selected from the group of carbapenems, cephalosporins, monobactams, penicillins, polypeptides, quinolones, sulfonamides and tetracyclines.

The terms "antibody", "immunoglobulin" and the like terms refer to a polypeptide substantially encoded by an immunoglobulin gene or immunoglobulin genes, or fragments thereof, which specifically bind and recognize an analyte (antigen). The recognized immunoglobulin genes include the kappa, lambda, alpha, gamma, delta, epsilon and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD, and IgE, respectively. An exemplary immunoglobulin (antibody) structural unit is composed of two pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (VL) and variable heavy chain (VH) refer to these light and heavy chains respectively. The C-terminal ends of each heavy chain are disulfide bonded together, and form the constant region of the antibody. Depending on the amino acid sequence of the constant domain of their heavy chains, antibodies can be assigned to different "classes". There are five-major classes of antibodies: IgA, IgD, IgE, IgG, and IgM, and several of these may be further divided into "subclasses" (isotypes), e.g., IgG1, IgG2, IgG3, IgG4, IgA, and IgA2. Full-length immunoglobulin "light chains" (of about 25 kDa or about 214 amino acids) comprise a variable region of about 1-10 amino acids at the NH2-terminus and a kappa or lambda constant region at the COOH-terminus. Full-length immunoglobulin "heavy chains" (of about 50 kDa or about 446 amino acids) similarly comprise a variable region (of about 1 16 amino acids) and one of the aforementioned heavy chain constant regions or classes, e.g., gamma (of about 330 amino acids). The subunit structures and three-dimensional configurations of different classes of immunoglobulins are well known.

In the context of the invention, the antibody is an anti-Gal antibody that recognizes and binds to a α-Gal epitope, wherein said epitope comprises a terminal α-galactosyl. More particularly, the anti-Gal antibody recognizes and binds to an agent comprising a terminal α-galactosyl moiety which is selected from the group comprising Galα1-3Gal, Galα1-2Gal, Galα1-6Gal, and α-galactose sugar unit(s) capable of binding anti-Gal antibodies. More preferably, the anti-Gal antibody recognizes and binds to an α-Gal epitope or to an agent comprising a terminal Galα1-3Gal. Even more preferably, the anti-Gal antibody recognizes and binds to GAS914. As used herein, the expression "an antibody targets" is referred to the specific recognition and binding of the antibody to a particular antigen. As it is used herein, the expression "binds specifically to" refers to the capacity of the antibodies for binding specifically to epitopes comprising a terminal α-galactosyl and not to epitopes comprising other carbohydrates.

The term "bacteria of the gastrointestinal tract", as used herein, also known as "intestinal microflora", or "gastrointestinal tract flora" relates to bacteria including aerobic and anaerobic bacteria, normally found in the intestinal tract in a healthy subject. The composition of the gastrointestinal tract flora differs between various animal species, and within an animal species. In humans, there are differences in the composition of the flora which are influenced by age, diet, cultural conditions, and the use of antibiotics. In the upper gastrointestinal tract of adult humans, the esophagus contains only the bacteria swallowed with saliva and food. Because of the high acidity of the gastric juice, very few bacteria (mainly acid-tolerant lactobacilli) can be cultured from the normal stomach. The proximal small intestine has a relatively sparse Gram-positive flora, consisting mainly of lactobacilli and *Enterococcus faecalis*. This region shows about 10⁵-10⁷ bacteria per ml of fluid. The distal part of the small intestine contains greater numbers of bacteria (10⁸/ml) and additional species, including coliforms *(E. coli* and relatives) and *Bacteroides,* in addition to lactobacilli and enterococci. The flora of the large intestine (colon) is qualitatively similar to that found in feces. Populations of bacteria in the colon reach levels of 10¹¹/ml feces. Coliforms become more prominent, and enterococci, clostridia and lactobacilli can be regularly found, but the predominant species are anaerobic *Bacteroides* and anaerobic lactic acid bacteria in the genus *Bifidobacterium (Bifidobacterium bifidum).* These organisms may outnumber *E. coli* by 1,000:1 to 10,000:1. Sometimes, significant numbers of anaerobic methanogens (up to 10¹⁰/gm) may reside in the colon of humans. The composition of the flora of the gastrointestinal tract varies along the tract (at longitudinal levels) and across the tract (at horizontal levels) where certain bacteria attach to the gastrointestinal epithelium and others occur in the lumen. In a particular embodiment of the invention, the bacteria of the gastrointestinal tract are enteric bacteria.

The term "enteric bacteria", as used herein, relates to Gram-negative rod-shaped bacteria with facultative anaerobic metabolism that live in the intestinal tracts of animals in health and disease. They are also characterized by being positive for true catalase and cytochromes, fermenting glucose by one of two major pathways to a variety of end products, being oxidase-negative, and possessing the enterobacterial common antigen in the cell wall. Some of the members of this family can live in the gut without causing health problems in individuals of good health, while others almost always cause signs of infection, including vomiting, diarrhea, and related symptoms. In a particular embodiment, enteric bacteria according to the invention are selected from the group consisting of bacteria of the genus *Acinetobacter, Actinomyces, Bacteroides, Bifidobacterium, Campylobacter, Clostridium, Corynebacterium, Enterococcus, Eubacterium, Fusobacterium, Haemophilus, Helicobacter, Lactobacilus, Mobiluncus, Peptostreptococcus, Porphyromonas, Prevotella, Propionibacterium, Pseudomonas, Staphylococcus, Streptococcus,* and *Veillonella,* and the Enterobacteriaceae family.

The term "Enterobacteriaceae family", as used herein, relates to enteric bacteria selected from the group consisting of bacteria of the genus *Citrobacter, Enterobacter, Escherichia, Klebsiella, Proteus, Salmonella, Serratia,* and *Yersinia.*

The term "epitope" or "antigenic determinant", as used herein, includes any region of an antigen which is specifically recognized by an antibody. One and the same antigen can have different epitopes. Each epitope usually consists of clusters of chemically active surfaces of molecules such as amino acids or sugar side chains, which have specific three-dimensional structural characteristics, as well as specific charge characteristics. In the context of the present invention, the epitope is an α-Gal epitope, wherein said epitope comprises a terminal α-galactosyl, and wherein said epitope is recognized and bound to by anti-Gal antibodies.

The term "infection", as used herein, relates to relates to invasion by bacteria, viruses, fungi, protozoa or other microorganisms, referring to the undesired proliferation or presence of invasion of pathogenic microbes in a host organism. It includes the excessive growth of microbes that are normally present in or on the body of a mammal or other organism. More generally, a microbial infection can be any situation in which the presence of a microbial population(s) is damaging to a host mammal. Thus, a microbial infection exists when excessive numbers of a microbial population are present in or on a mammal's body, or when the effects of the presence of a microbial population(s) is damaging the cells or other tissue of a mammal. In particular, in the context of the present invention, the infection is a bacterial infection. In a particular embodiment, the bacterial infection is caused by bacteria of the gastrointestinal tract. In a more particular embodiment, the bacteria of the gastrointestinal tract is an enteric bacteria, preferably an enteric bacteria selected from the group consisting of bacteria of the genus *Acinetobacter, Actinomyces, Bacteroides, Bifidobacterium, Campylobacter, Clostridium, Corynebacterium, Enterococcus, Eubacterium, Fusobacterium, Haemophilus, Helicobacter, Lactobaacilus, Mobiluncus, Peptostreptococcus, Porphyromonas, Prevotella, Propionibacterium, Pseudomonas, Staphylococcus, Streptococcus,* and *Veillonella,* and the Enterobacteriaceae family.

The term "galactose" or "Gal", as used herein, relates to a C-4 epimer of glucose that exists in both open chain and cyclic form. The open-chain form has a carbonyl at the end of the chain. In the open-chain form D- and L- isomers cannot be separated, but the cyclic forms can be crystallized and isolated.

The term "Gal(α1,3)Gal" or "Gal(α1,3)" or α1,3 galactobiose (in some contexts, also referred to as alpha-Gal) relates to galactose(α1,3)galactose (CAS No 13168-24-6). Compounds comprising a galactose-α-1,3-galactose epitope include, without limitation, galactose-α-1,3-galactose and derivatives thereof including oligomers (e.g., dimers, trimers, tetramers, pentamers) of galactose-α-1,3-galactose and glycopeptides bearing oligosaccharides with terminal galactose-α-1,3-galactose. Derivatives of galactose-α-1,3-galactose include, for example, amides, esters, ethers, amines, sulfonamides, thioethers, acetals, carbamates, ureas and amidines. Examples of compounds comprising a galactose-α-1,3-galactose epitope, by way of illustration and not limitation, include galactose-α-1,3-galactose (α1-3 galactobiose) (Galα1-3Gal) and derivatives thereof such as, for example, glucosamine derivatives, linear B-2 trisaccharide (Galα1-3Galβ1-4GlcNAc), linear B-6 trisaccharide (Galα1-3Galβ1-4Glc) and derivatives thereof, α1-3 galactobiosyl β-methyl glycoside, α1-3, β1-4 galactotriose (Galα1-3Galβ1-4Gal), galactotetraose (Galα-3Galβ1-4Galα1-3-D-Gal), and derivatives of the above such as, e.g., amino acid derivatives (e.g., dodecalysine, glycine) and glycopeptides with galactose-a-1,3-galactose.

Similarly, the terms "Gal(α1,2)Gal", "Gal(α1,6)Gal" and "Gal(α1,6)Glc" relate, respectively, to galactose(α1,2)galactose, galactose(α1,6)galactose and galactose(α1,6)glucose.

The term "GAS914", as used herein, relates to a soluble Gal trisaccharide-polylysine conjugate of approximately 500 kDa that effectively competes for α-Gal binding by α-Gal IgM (IC(50), 43 nM) and IgG (IC(50), 28 nM) *antibodies in vitro* (Katopodis G et al. 2002 J. Clin. Invest., 110: 1869-1877; Zhong R et al. 2003 Transplantation 75: 10-19). It is an artificial injectable antigen with a linear polylysine backbone (with an average length of 1,000 lysines) and with approximately 25% of side chains conjugated to Linear B trisaccharide (linear B type 2 trisaccharide, Galα1-3Galβ1-4GlcNAc). It is described in WO9847915 by Novartis AG (Basel, Switzerland). GAS914 has the following structure (Katopodis G et al. 2002 J Clin Invest 110(12):1869-1877).

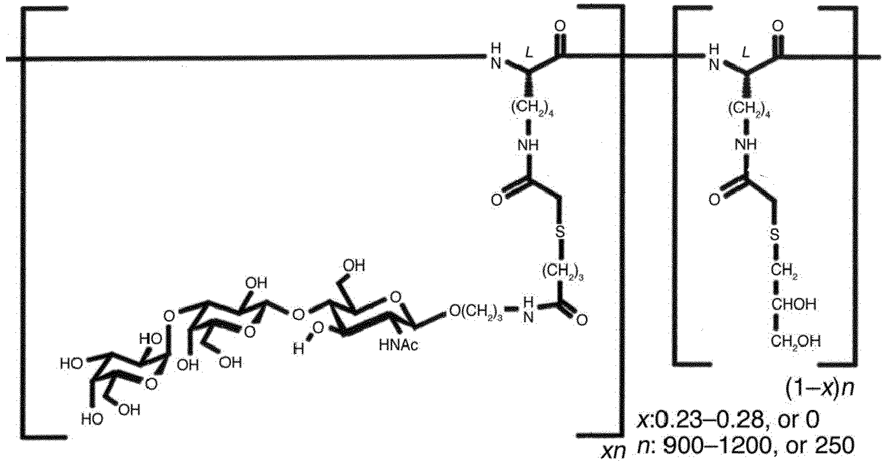

GAS914 is shown herein as the chemical structure of random copolymer, wherein n represents the average degree of polymerization; x represents the fraction of glycosylated monomer; and 1 - x represents the fraction of thioglycerol-capped monomer.

The term "glycoconjugate", as used herein, relates to carbohydrates covalently linked with a second chemical species, wherein said second chemical species is selected from the group comprising proteins (known as glycoproteins), peptides (peptidoglycans, glucopeptides), lipids (glycolipids, lipopolisaccharides), saccharides (glycosaccharides). Glycoconjugates according to the invention include, without limitation, those wherein a carbohydrate comprising a terminal alpha-galactosyl moiety is conjugated to bovine serum albumin (BSA), human serum albumin (HSA), or 1,2-di-O-hexadecyl-sn-glycero-3-phosphoethanolamine (HDPE).

The term "glycolipid" relates to a lipid molecule attached to a carbohydrate. Glycolipids comprise glyceroglycolipids, glycosphingolipids and glycosylphosphatidylinositols. Glycolipids according to the invention comprise at least a carbohydrate residue comprising a terminal alpha-galactosyl residue. In a particular embodiment, glycolipids according to the invention comprise a terminal Galα1-3Gal, terminal Galα1-2Gal, terminal Galα1-6Gal, terminal Galα1-6Glc, or terminal α-galactose sugar unit(s) capable of binding an anti-Gal antibody, more preferably a terminal Galα1-3Gal.

The term "glycoprotein" relates to any protein which is covalently modified by at least one carbohydrate residue. Glycoproteins which can be studied according to the method of the invention include those which are modified by a monosaccharide or by an oligosaccharide and in which said monosaccharide or oligosaccharide is bound to the polypeptide chain by a side chain comprising a nitrogen atom (N-glycosylation) or an oxygen atom (O-glycosylation). Typically, N-glycosylation comprises the modification of proteins in asparagine residues which form part of a consensus sequence of the Asn-X-Ser or Asn-X-Thr type. O-glycosylation occurs in the side chains of serine and/or threonine residues. Glycoproteins and glycopeptides according to the invention comprise at least a carbohydrate residue comprising a terminal α-galactosyl moiety, in particular selected from the group comprising a terminal Galα1-3Gal, terminal Galα1-2Gal, terminal Galα1-6Gal, terminal Galα1-6Glc, or terminal α-galactose sugar unit(s) capable of binding an anti-Gal antibody, more preferably a terminal Galα1-3Gal.

The term "moiety", as used herein, relates to a part of a molecule that may include either whole functional groups or parts of functional groups as substructures.

The term "subject" or "individual" or "animal" or "patient" is meant any subject, particularly a mammalian subject, for whom diagnosis, prognosis, or therapy is desired. Mammalian subjects include humans, domestic animals, farm animals, and zoo, sports, or pet animals such as dogs, cats, guinea pigs, rabbits, rats, mice, horses, cattle, cows, and so on. In a preferred embodiment of the invention, the subject lacks α-Gal epitopes but has natural anti-Gal antibodies, and includes humans, apes, and Old World monkeys (superfamily *Cercopithecoidea,* clade *Catarrhini,* including baboons and macaques). In a more preferred embodiment of the invention, the subject is a human. In a particular embodiment, the subject suffers from an infection, in particular the subject suffers from an infection caused by bacteria of the gastrointestinal tract, more particularly caused by an enteric bacteria.

### Agents for use in the prevention and/or treatment of infection

In a first aspect, the invention relates to an agent comprising a terminal α-galactosyl moiety for use in the prevention and/or treatment of infection in a subject, wherein said infection is caused by bacteria of the gastrointestinal tract.

Alternatively, the invention relates to the use of an agent comprising a terminal α-galactosyl moiety, for the manufacture of a medicament for the prevention and/or treatment of infection in a subject, wherein said infection is caused by bacteria of the gastrointestinal tract. Alternatively, the invention relates to a method for the prevention and/or treatment of infection in a subject, wherein said infection is caused by bacteria of the gastrointestinal tract, that comprises administering to a subject in need thereof a therapeutically effective amount of an agent comprising a terminal α-galactosyl moiety.

Thus, the invention relates to agents for use in the prevention and/or treatment of infection in a subject comprising a terminal α-galactosyl moiety. Said infection to be prevented and/or treated by the agent for use of the invention is caused by bacteria of the gastrointestinal tract. In a particular embodiment, the bacteria of the gastrointestinal tract are enteric bacteria. More particularly, the enteric bacteria are selected from the group consisting of bacteria of the genus *Acinetobacter, Actinomyces, Bacteroides, Bifidobacterium, Campylobacter, Clostridium, Corynebacterium, Enterococcus, Eubacterium, Fusobacterium, Haemophilus, Helicobacter, Lactobacilus, Mobiluncus, Peptostreptococcus, Porphyromonas, Prevotella, Propionibacterium, Pseudomonas, Staphylococcus, Streptococcus* and *Veillonella,* and the Enterobacteriaceae family. More particularly, the enteric bacteria of the Enterobacteriaceae family are selected from the group consisting of bacteria of the genus *Citrobacter, Enterobacter, Escherichia, Klebsiella, Proteus, Salmonella, Serratia, and Yersinia.*

*Acinetobacter* is a genus of aerobic, Gram-negative bacteria belonging to the wider class of *Gammaproteobacteria. Acinetobacter* species are not motile and oxidase-negative, and occur in pairs under magnification. *Acinetobacter* species are a key source of infection in debilitated patients in the hospital, in particular the species *Acinetobacter baumannii.* In a particular embodiment, the *Acinetobacter* enteric bacteria is *Acinetobacter baumannii.*

*Actinomyces* is a genus of Gram-positive actinobacteri, facultatively anaerobic (except *A. meyeri,* a strict anaerobe). All species grow best under anaerobic conditions. *Actinomyces* species do not form endospores, and, while individual bacteria are rod-shaped, *Actinomyces* colonies form fungus-like branched networks of hyphae. Actinomyces species are normally present in the gums and are the most common cause of infection in dental procedures and oral abscesses. Many *Actinomyces* species are opportunistic pathogens of humans and other mammals, particularly in the oral cavity. In rare cases, these bacteria can cause actinomycosis, a disease characterized by the formation of abscesses in the mouth, lungs, or the gastrointestinal tract. Actinomycosis is most frequently caused by *Actinomyces israelii. A. israelii* may also cause endocarditis.

*Bacteroides* is a genus of Gram-negative, obligately anaerobic bacteria. *Bacteroides* species are non-endospore-forming bacilli, and may be either motile or non-motile, depending on the species. The DNA base composition is 40-48% GC. Some species (*B. fragilis,* for example) are opportunistic human pathogens, causing infections of the peritoneal cavity, gastrointestinal surgery, and appendicitis via abscess formation, inhibiting phagocytosis, and inactivating beta-lactam antibiotics. Although *Bacteroides* species are anaerobic, they are transiently aerotolerant and thus can survive in the abdominal cavity.

*Bifidobacterium* is a genus of Gram-positive, non-motile, often branched anaerobic bacteria. They are ubiquitous, endosymbiotic inhabitants of the gastrointestinal tract, vagina and mouth (*B. dentium)* of mammals, including humans. Bifidobacteria are one of the major genera of bacteria that make up the colon flora in mammals. Some bifidobacteria are used as probiotics.

*Campylobacter* is a genus of microaerophilic Gram-negative bacteria. It is a significant cause of food poisoning due to handling raw meat or undercooking it. Motile, with either unipolar or bipolar flagella, the organisms have a characteristic spiral/corkscrew appearance and are oxidase-positive. *Campylobacter jejuni* is one of the main causes of bacterial foodborne disease in many developed countries. At least a dozen species of *Campylobacter* have been implicated in human diseases. Preferred embodiments of the invention include C. *jejuni* and C. *coli.* Campylobacteriosis relates to the infection caused by *Campylobacter.* It produces an inflammatory, sometimes bloody, diarrhea, periodontitis or dysentery syndrome, mostly including cramps, fever and pain.

*Clostridium* is a genus of Gram-positive bacteria, which are obligate anaerobes capable of producing endospores. Individual cells are rod-shaped. There are five main species responsible for disease in humans, all of which are preferred embodiments of the invention: *C. botulinum* (it produces botulinum toxin in food/wound and can cause botulism), *C. difficile* (it can flourish when other bacteria in the gut are killed during antibiotic therapy, leading to pseudomembranous colitis, a cause of antibiotic-associated diarrhea), *C. perfringens* (also known as *C. welchii,* it causes a wide range of symptoms, from food poisoning to gas gangrene, and is also responsible for enterotoxemia), *C. tetani* (it is the causative organism of tetanus) and *C. sordellii* (it may cause a fatal infection in exceptionally rare cases after medical abortions).

*Corynebacterium* is a genus of Gram-positive, rod-shaped bacteria, widely distributed in nature and mostly innocuous. In a preferred embodiment, the *Corynebacterium* is *C. diphtheriae.*

*Enterococcus* is a genus of Gram-positive, lactic acid bacteria of the phylum Firmicutes. Important clinical infections caused by *Enterococcus* include urinary tract infections, bacteremia, bacterial endocarditis, diverticulitis, and meningitis. Sensitive strains of these bacteria can be treated with ampicillin, penicillin and vancomycin. In a particular embodiment, the *Enterococcus* enteric bacteria are selected from the group consisting of *Enterococcus faecalis and Enterococcus faecium.*

*Eubacterium* is a genus of either Gram-positive or Gram-negative bacteria genus in the family Eubacteriaceae. These bacteria are characterised by a rigid cell wall. They may either be motile (they have a flagellum) or non-motile.

*Fusobacterium* is a genus of anaerobic, Gram-negative bacteria, wherein individual cells are rod-shaped bacilli with pointed ends.

*Haemophilus* is a genus of Gram-negative, pleomorphic, coccobacilli bacteria belonging to the Pasteurellaceae family, and either aerobic or facultatively anaerobic. Preferred embodiment include *H. influenza* and *H. ducreyi* (the causative agent of chancroid).

*Helicobacter* is a genus of Gram-negative bacteria having a characteristic helix shape. In a preferred embodiment, the Helicobacter bacteria is *H. pylori.*

*Lactobacilus* is a genus of Gram-positive facultative anaerobic or microaerophilic rod-shaped bacteria.

*Mobiluncus* is a genus of gram-positive, anaerobic, rod-shaped bacteria. They are found in the human vagina, particularly in association with *Gardnerella vaginalis* in cases of bacterial vaginosis.

*Peptostreptococcus* is a genus of anaerobic, Gram-positive, non-spore forming bacteria. In one embodiment, the bacteria of the *Peptostreptococcus* genus is *P. magnus.*

*Porphyromonas* is a genus of non-motile, Gram-negative, rod-shaped, anaerobic bacteria. In one embodiment, the bacteria of the *Porphyromonas* is *P. gingivalis.*

*Prevotella* is a genus of Gram-negative bacteria. *Prevotella* spp. are members of the oral and vaginal flora and are recovered from anaerobic infections of the respiratory tract.

*Propionibacterium* is a Gram-positive, rod-shaped genus of bacteria able to synthesize propionic acid by transcarboxylase enzymes.

*Pseudomonas* is a genus of Gram-negative aerobic gammaproteobacteria. In a particular embodiment, the *Pseudomonas* enteric bacteria are *Pseudomonas aeruginosa.*

*Staphylococcus* is a genus of Gram-positive bacteria, with round appearance (cocci) and form in grape-like clusters. In a particular embodiment, the *Staphylococcus* enteric bacteria are selected from the group consisting of *Staphylococcus aureus.*

*Streptococcus* is a genus of spherical Gram-positive bacteria belonging to the phylum Firmicutes and the lactic acid bacteria group.

*Veillonella* is a genus of Gram-negative anaerobic bacteria, known for its lactate fermenting abilities. In a preferred embodiment, the bacteria of the genus *Veillonella* is *Veillonella parvula.*

Enterobacteriaceae is a large family of Gram-negative bacteria that includes harmless symbionts as well as pathogens (including *Salmonella, Escherichia coli, Yersinia pestis, Klebsiella* and *Shigella).* Other disease-causing bacteria in this family include *Proteus, Enterobacter, Serratia,* and *Citrobacter.* Many members of this family are a normal part of the gut flora found in the intestines of humans and other animals, while others are found in water or soil, or are parasites on a variety of different animals and plants. *Escherichia coli* is one of the most important model organisms. In a particular embodiment, the enteric bacteria of the Enterobacteriaceae family is selected from the group consisting of bacteria of the genus *Citrobacter, Enterobacter, Escherichia, Klebsiella, Proteus, Salmonella, Serratia* and *Yersinia.*

*Citrobacter* is a genus of Gram-negative coliform bacteria in the Enterobacteriaceae family. The species *C. amalonaticus, C. koseri,* and *C. freundii* can use citrate as a sole carbon source. These species are all preferred embodiments of the invention.

*Enterobacter* is a genus of common Gram-negative, facultatively anaerobic, rod-shaped, non-spore-forming bacteria of the family Enterobacteriaceae. In a particular embodiment, the *Enterobacter* enteric bacteria are selected from the group consisting of *Enterobacter aerogenes* and *Enterobacter cloacae.*

*Escherichia* is a genus of Gram-negative, non-spore forming, facultatively anaerobic, rod-shaped bacteria from the family Enterobacteriaceae. In a particular embodiment, the *Escherichia* enteric bacteria are *Escherichia coli.*

*Klebsiella* is a genus of non-motile, Gram-negative, oxidase-negative, rod-shaped bacteria with a prominent polysaccharide-based capsule, from the family Enterobacteriaceae. In a particular embodiment, the *Klebsiella* enteric bacteria are *Klebsiella pneumoniae*

*Proteus* is a genus of Gram-negative Proteobacteria, from the family Enterobacteriaceae. In a preferred embodiment, the bacteria of the genus Proteus is *P. mirabilis* or *P*.*vulgaris*.

*Salmonella* is a genus of rod-shaped, Gram-negative bacteria, from the family Enterobacteriaceae. There are only two species of *Salmonella, Salmonella bongori* and *Salmonella enterica,* of which there are around six subspecies. Salmonellae are found worldwide in both cold-blooded and warm-blooded animals, and in the environment. They cause illnesses such as typhoid fever, paratyphoid fever, and food poisoning. *Salmonella* species are facultative intracellular pathogens. Many infections are due to ingestion of contaminated food. They can be divided into two groups, typhoidal and nontyphoidal, *Salmonella* serovars. Typhoidal serovars include *Salmonella typhi* and *Salmonella paratyphi.*

*Yersinia* is a genus of Gram-negative rod shaped bacteria in the family Enterobacteriaceae, which are a few micrometers long and fractions of a micrometer in diameter, and are facultative anaerobes. Some members of *Yersinia* are pathogenic in humans; in particular, *Y. pestis* is the causative agent of the plague. Rodents are the natural reservoirs of *Yersinia* and, less frequently, other mammals serve as the host. Infection may occur either through blood (in the case of *Y. pestis)* or in an alimentary fashion, occasionally via consumption of food products (especially vegetables, milk- derived products, and meat) contaminated with infected urine or feces.

In a particular embodiment, the subject suffering from infection by bacteria of the gastrointestinal tract, or candidate to suffer from infection by bacteria of the gastrointestinal tract, has natural endogenous anti-Gal antibodies. In a particular preferred embodiment, the subject is a human.

The infection caused by bacteria of the gastrointestinal tract, particularly enteric bacteria, may occur in any organ or tissue of the subject. In a particular embodiment, the infection caused by bacteria of the gastrointestinal tract, particularly enteric bacteria, occurs in blood, gastrointestinal tract, heart, cardiovascular system, liver, lung, respiratory tract, kidney, urinary tract, nervous central system, skin, subcutaneous tissues or surgical wounds. In a more particular embodiment, the infection caused by bacteria of the gastrointestinal tract, particularly enteric bacteria, occurs in blood.

In one embodiment, the therapeutic effect of the agent comprising a terminal α-galactosyl moiety is achieved by depleting anti-Gal antibodies from serum and enhancing the bactericidal activity of the serum. In another embodiment, the therapeutic effect of the agent comprising the terminal α-galactosyl moiety is not achieved by modulating the inflammatory response of the patient against the bacteria causing the infection.

As previously described, the agent for use according to the invention comprises a terminal α-galactosyl moiety. In a particular embodiment, the terminal α-galactosyl moiety is selected from the group comprising terminal Galα1-3Gal, terminal Galα1-2Gal, terminal Galα1-6Gal, terminal Galα1-6Glc, and terminal α-galactose sugar unit(s) capable of binding an anti-Gal antibody. In a particular preferred embodiment, the terminal α-galactosyl moiety of the agent for use according to the invention comprises a terminal Galα1-3Gal.

Natural or synthetic molecules comprising one or more terminal α-galactosyl moietys according to the present invention include, without limitation, the following:
i. galactose-α-1,3-galactose, oligomers (e.g., dimers or disaccharides, trimmers or trisaccharides, tetramers or tetrasaccharides, pentamers or pentasaccharides) of galactose-α-1,3-galactose, and derivatives thereof, including glucosamine derivatives, linear B-2 trisaccharide (Galα1-3Galβ1-4GlcNAc, CAS No. 101627-01-4), linear B-6 trisaccharide (Galα1-3Galβ1-4Glc, CAS No. 56038-36-9) and derivatives thereof, α1-3 galactobiosyl β-methyl glycoside; α1-3, β1-4 galactotriose (Galα1-3Galβ1-4Gal, CAS No. 56038-36-9), galactotetraose (Galα1-3Galβ1-4Galα1-3-D-Gal, CAS No. 56038-38-1), Galili pentasaccharide (L537, Gal-α1,3Gal-β1,4GlcNAc-β1,3Gal-β1,4Glc, CAS No. 119502-59-9), and derivatives of the above such as, e.g., amino acid derivatives (e.g., dodecalysine, glycine), amides, esters, ethers, amines, sulfonamides, thioethers, acetals, carbamates, ureas and amidines,
ii. glycopeptides comprising oligosaccharides with terminal galactose-α-1,3-galactose,
iii. commercially available glycolipids (Dextra Laboratories, Ltd., United Kingdom) such as Galα1-3Gal glycolipids comprising blood group B type 2 linear trisaccharide (GN334, Galα1-3Galβ1-4GlcNac, CAS No. 101627-01-4); and comprising Galili pentasaccharide (L537, Gal-α1,3Gal-β1,4GlcNAc-β1,3Gal-β1,4Glc, CAS No. 119502-59-9),
iv. commercially available glycoconjugates with galactose-α-1,3-galactose epitopes include for instance: Galα1-3Galβ1-4Glc-BSA (#NGP0330, 3 atom spacer), Galα1-3Galβ1-4(3-deoxyGlcNAc)-HSA (#NGP2335), Galα1-3Galβ1-4GlcNAcβ1-HDPE (#NGL0334), Galα1-3Gal-BSA (#NGP0203, 3 atom spacer), Galα1-3Galβ1-3GlcNAc-BSA (#NGP0333, 3 atom spacer), Galα1-3Galβ1-3GlcNAc-HSA (#NGP2333, 3 atom spacer), Galα1-3Galβ1-4(6-deoxyGlcNAc)-HSA (#NGP2336, 3 atom spacer), Galα1-3Galβ1-4Glc-HSA (#NGP2330, 3 atom spacer), Galα1-3Gal-BSA (#NGP1203, 14 atom spacer), Galα1-3Gal-HSA (#NGP2203, 3 atom spacer), Galα1-3Gal-HSA (#NGP3203, 14 atom spacer), Galα1-3Galβ1-4GlcNAc-BSA (#NGP0334, 3 atom spacer), Galα1-3Galβ1-4GlcNAc-BSA (#NGP1334, 14 atom spacer), Galα1-3Galβ1-4GlcNAc-HSA (#NGP2334, 3 atom spacer), Galα1-3Galβ1-4GlcNAc-HSA (#NGP3334, 14 atom spacer), and Galα1-3Galβ1-4Glc-BSA (#NGP0330, 3 atom spacer), Galal-3Galβ1-HDPE (#NGL0203),
v. galactose-α-1,3-galactose oligosaccharides, such as those commercially available from Elicityl, including without limitation Galα1-3Galβ1-3(Fucα1-4)GlcNAc (#GLY076), Galα1-3Galβ1-4(Fucα1-3)GlcNAc (#GLY075), Galα1-3[Galβ1-4GlcNAcβ1-3]4Galβ1-4Glc (#GLY079), Galα1-3[Galβ1-4GlcNAcβ1-3]3Galβ1-4Glc (#GLY078), Galα1-3Galβ1-4Glc (#GLY070), Galα1-3[Galβ1-4GlcNAcβ1-3]2Galβ1-4Glc (#GLY077), Galα1-3Galβ1-4GlcNAcβ1-3Galβ1-4Glc (#GLY071), Galα1-3Galβ1-4GlcNAc (#GLY74-2, linear B-2 trisaccharide), and Galα1-3Galβ1-3GlcNAc (#GLY74-1), and
vi. additional macromolecules with α-Gal epitopes that are suitable for injection and *subsequent in situ* binding to anti-Gal antibodies include, without limitation:
   o mouse laminin with 50-70 α-gal epitopes (Galili U 1993 Springer Seminars in Immunopathology 15, 155), commercially available from Life Technologies (#23017-015),
   o multiple synthetic α-gal epitopes linked to BSA (Stone KR et al. 2007 Transplantation 83(2): 211-219),
   o GAS914, produced by Novartis (WO9847915) and disclosed in Zhong R et al. 2003 Transplantation 75(1): 10-19,
   o the α-Gal polyethylene glycol conjugate TPC (Schirmer JM et al. 2004 Xenotransplantation 11(5): 436-443), and
   o α-Gal epitope-mimicking peptides linked to a macromolecule backbone (Sandrin MS et al. 1997 Glycoconj J 14(1): 97-105), and peptides encoded by mucin genes MUC1, 3 and 4.

These compounds, and similar synthetic and natural glycoconjugates, glycolipids, glycoproteins or oligosaccharides linked to reactive groups comprising a terminal α-galactosyl moiety, preferably a terminal Galα1-3Gal, may also bind anti-Gal antibodies and thus be considered functional equivalents of the agents for their use according to the invention. In a particular preferred embodiment, the agent comprising a terminal α-galactosyl moiety for use according to the invention is GAS914.

Assays to determine whether a molecule is capable of specifically binding to an antibody, particularly an anti-Gal antibody, are known by the skilled person and include, without limitation, immunoprecipitation, radioimmunoassay (RIA), enzyme-linked immunoabsorbent assay (ELISA) and immunofluorescent techniques such as fluorescence microscopy or flow cytometry.

Similarly, methods to determine the bactericidal activity of an agent are known by the skilled person and include the assay as performed by the inventors and described in Example 2 of the present application, as well as those assays to determine the minimum bactericidal concentration (MBC) of a drug and the serum bactericidal titer (SBT) of a patient's blood or body fluid during treatment as described by Hacek (Hacek DM et al. 1999 J Clin Microbiol 37(6): 1881-1884).

Appropriate amounts of an agent according to the present invention can be formulated with pharmaceutically acceptable excipients and/or carriers to obtain a pharmaceutical composition for use in the prevention and/or treatment of infection caused by bacteria of the gastrointestinal tract. A composition that includes an agent according to the invention can be delivered to a subject by a variety of routes including, without limitation, systemically delivery, e.g., by intravenous, subcutaneous, intramuscular injection. Additionally, it is also possible to administer the composition comprising the agent of the invention intranasally or sublingually which allows systemic administration by a non-aggressive mode of administration. Also, intraventricular administration may be adequate. A preferred route of delivery is subcutaneous injection.

Those skilled in the art are familiar with the principles and procedures discussed in widely known and available sources as Remington's Pharmaceutical Science (17th Ed., Mack Publishing Co., Easton, Pa., 1985) and Goodman and Gilman's The Pharmaceutical Basis of Therapeutics (8th Ed., Pergamon Press, Elmsford, N.Y., 1990) both of which are incorporated herein by reference.

In a preferred embodiment of the present invention, the agents of the invention are formulated in accordance with standard procedure as a pharmaceutical composition adapted for delivered administration to human beings and other mammals producing natural anti-Gal antibodies. Typically, compositions for intravenous, intramuscular, subcutaneous or intraventricular administration are solutions in sterile isotonic aqueous buffer.

Where necessary, the agent of the invention is comprised in a composition also including a solubilizing agent and a local anesthetic to ameliorate any pain at the site of the injection. Generally, the ingredients are supplied either separately or mixed together in unit dosage form, for example, as a dry lyophilized powder or water free concentrate in a hermetically sealed container such as an ampule or sachette indicating the quantity of active agent. Where the composition is to be administered by infusion, it can be dispensed with an infusion bottle containing sterile pharmaceutical grade water or saline. Where the composition is administered by injection, an ampule of sterile water for injection or saline can be provided so that the ingredients may be mixed prior to administration.

In cases other than intravenous administration, the composition can contain minor amounts of wetting or emulsifying agents, or pH buffering agents. The composition can be a liquid solution, suspension, emulsion, gel, polymer, or sustained release formulation. The composition can be formulated with traditional binders and carriers, as would be known in the art. Formulations can include standard carriers such as pharmaceutical grades of mannitol, lactose, starch, magnesium stearate, sodium saccharide, cellulose, magnesium carbonate, etc., inert carriers having well established functionality in the manufacture of pharmaceuticals. Various delivery systems are known and can be used to administer a therapeutic of the present invention including encapsulation in liposomes, microparticles, microcapsules and the like.

In yet another preferred embodiment, therapeutics containing the agents of the invention can be formulated as neutral or salt forms. Pharmaceutically acceptable salts include those formed with free amino groups such as those derived from hydrochloric, phosphoric, acetic, oxalic, tartaric acids and the like, and those formed with free carboxyl groups such as those derived from sodium, potassium, ammonium, calcium, ferric hydroxides, isopropylamine, thriethylamine, 2-ethylamino ethanol, histidine, procaine or similar.

The pharmaceutical compositions containing the agent according to the invention can occur at any pharmaceutical form of administration considered appropriate for the selected administration route, for example, by systemic, oral, parenteral or topical administration, for which it will include the pharmaceutically acceptable excipients necessary for formulation of the desired method of administration.

The effective quantity of the agent of the invention can vary within a wide range and, in general, will vary depending on the particular circumstances of application, duration of the exposure and other considerations. In a particular embodiment, the dose ranges between 0.05 mg/kg and 20 mg/kg.

Solid dosage forms for oral administration may include conventional capsules, sustained release capsules, conventional tablets, sustained-release tablets, chewable tablets, sublingual tablets, effervescent tablets, pills, suspensions, powders, granules and gels. At these solid dosage forms, the active compounds can be mixed with at least one inert excipient such as sucrose, lactose or starch. Such dosage forms can also comprise, as in normal practice, additional substances other than inert diluents, e.g. lubricating agents such as magnesium stearate. In the case of capsules, tablets, effervescent tablets and pills, the dosage forms may also comprise buffering agents. Tablets and pills can be prepared with enteric coatings.

Liquid dosage forms for oral administration may include emulsions, solutions, suspensions, syrups and elixirs pharmaceutically acceptable containing inert diluents commonly used in the technique, such as water. Those compositions may also comprise adjuvants such as wetting agents, emulsifying and suspending agents, and sweetening agents, flavoring and perfuming agents.

Injectable preparations, for example, aqueous or oleaginous suspensions, sterile injectable may be formulated according with the technique known using suitable dispersing agents, wetting agents and/or suspending agents. Among the acceptable vehicles and solvents that can be used are water, Ringer's solution and isotonic sodium chloride solution. Sterile oils are also conventionally used as solvents or suspending media.

For topical administration, compounds of the invention can be formulated as creams, gels, lotions, liquids, pomades, spray solutions, dispersions, solid bars, emulsions, microemulsions and similars which may be formulated according to conventional methods that use suitable excipients, such as, for example, emulsifiers, surfactants, thickening agents, coloring agents and combinations of two or more thereof.

Additionally, the compounds of the invention may be administered in the form of transdermal patches or iontophoresis devices. In one embodiment, the compounds of the invention are administered as a transdermal patch, for example, in the form of sustained-release transdermal patch. Suitable transdermal patches are described in more detail in, for example, US5262165, US5948433, US6010715 and US6071531.

The compositions comprising the agents of the invention can additionally include conventional excipients, ie pharmaceutically acceptable carriers suitable for parenteral application which do not react damaging with the active compounds. Suitable pharmaceutically acceptable vehicles include, for example, water, salt solutions, alcohol, vegetable oils, polyethylene glycols, gelatin, lactose, amylose, magnesium stearate, talc, surfactants, silicic acid, viscous paraffin, perfume oil, monoglycerides and diglycerides of fatty acids, fatty acid esters petroetrals, hydroxymethyl cellulose, polyvinylpyrrolidone and similars.

Several drug delivery systems are known and can be used to administer the agents or compositions of the invention, including, for example, encapsulation in liposomes, microbubbles, emulsions, microparticles, microcapsules and similars. The required dosage can be administered as a single unit or in a sustained release form.

Sustainable-release forms and appropriate materials and methods for their preparation are described in, for example, "Modified-Release Drug Delivery Technology", Rathbone, M. J. Hadgraft, J. and Roberts, M. S. (eds.), Marcel Dekker, Inc., New York (2002), "Handbook of Pharmaceutical Controlled Release Technology", Wise, D. L. (ed.), Marcel Dekker, Inc. New York, (2000). In one embodiment of the invention, the orally administrable form of a compound according to the invention is in a sustained release form further comprises at least one coating or matrix. The coating or sustained release matrix include, without limitation, natural polymers, semisynthetic or synthetic water-insoluble, modified, waxes, fats, fatty alcohols, fatty acids, natural semisynthetic or synthetic plasticizers, or a combination of two or more of the them.

Enteric coatings may be applied using conventional processes known to experts in the art, as described in, for example, Johnson, J. L., "Pharmaceutical tablet coating", Coatings Technology Handbook (Second Edition), Satas, D. and Tracton, A. A. (eds), Marcel Dekker, Inc. New York, (2001), Carstensen, T., "Coating Tablets in Advanced Pharmaceutical Solids", Swarbrick, J. (ed.), Marcel Dekker, Inc. New York (2001), 455-468.

Even though individual needs vary, determination of optimal ranges for effective amounts of the agent of the invention belongs to the common experience of those experts in the art. In general, the dosage needed to provide an effective amount of such compound, which can be adjusted by one expert in the art will vary depending on age, health, fitness, sex, diet, weight, degree of alteration of the receptor, frequency of treatment and the nature and extent of impairment or illness, medical condition of the patient, route of administration, pharmacological considerations such as activity, efficacy, pharmacokinetic and toxicology profile of the particular compound used, if using a system drug delivery, and if the compound is administered as part of a combination of drugs.

The amount of the compound according to the invention that is effective in the treatment of a particular disorder or condition will depend on the nature of the disorder or condition, in particular an infection caused by bacteria of the gastrointestinal tract, and can be determined by conventional clinical techniques, including reference to Goodman and Gilman, supra; The Physician's Desk Reference, Medical Economics Company, Inc., Oradell, NJ, 1995, and Drug Facts and Comparisons, Inc., St. Louis, MO, 1993. The precise dose to use in the formulation will also depend on the route of administration, and severity of the disease or disorder, and should be decided in the physician's opinion and the patient's circumstances.

### Compositions of the invention and uses thereof

In another aspect, the invention relates to a composition comprising
(i) an agent comprising a terminal α-galactosyl moiety, and
(ii) an antibiotic.

Agents comprising a terminal α-galactosyl moiety according to the invention, have been described previously. In a particular embodiment, the terminal α-galactosyl is selected from the group comprising terminal Galα1-3Gal, terminal Galα1-2Gal, terminal Galα1-6Gal, terminal Galα1-6Glc, and terminal α-galactose sugar unit(s) capable of binding an anti-Gal antibody. In a more particular embodiment, the terminal α-galactosyl is terminal Galα1-3Gal. In a preferred embodiment, the agent is selected from the following:
- galactose-α-1,3-galactose, oligomers (e.g., dimers, trimmers, tetramers, pentasaccharides) of galactose-α-1,3-galactose, and derivatives thereof, including glucosamine derivatives, linear B-2 trisaccharide (Galα1-3Galβ1-4GlcNAc, CAS No. 101627-01-4), linear B-6 trisaccharide (Galα1-3Galβ1-4Glc, CAS No. 56038-36-9) and derivatives thereof, α1-3 galactobiosyl β-methyl glycoside; α1-3, β1-4 galactotriose (Galα1-3Galβ1-4Gal, CAS No. 56038-36-9), galactotetraose (Galα1-3Galβ1-4Galα1-3-D-Gal, CAS No. 56038-38-1), Galili pentasaccharide (L537, Gal-α1,3Gal-β1,4GlcNAc-β1,3Gal-β1,4Glc, CAS No. 119502-59-9), and derivatives of the above such as, e.g., amino acid derivatives (e.g., dodecalysine, glycine), amides, esters, ethers, amines, sulfonamides, thioethers, acetals, carbamates, ureas and amidines,
- glycopeptides comprising oligosaccharides with terminal galactose-α-1,3-galactose,
- commercially available glycolipids (Dextra Laboratories, Ltd., United Kingdom) such as Galα1-3Gal glycolipids comprising blood group B type 2 linear trisaccharide (GN334, Galα1-3Galβ1-4GlcNac, CAS No. 101627-01-4); and comprising Galili pentasaccharide (L537, Gal-α1,3Gal-β1,4GlcNAc-β1,3Gal-β1,4Glc, CAS No. 119502-59-9),
- commercially available glycoconjugates with galactose-α-1,3-galactose epitopes available from Dextra include for instance: Galα1-3Galβ1-4Glc-BSA (#NGP0330, 3 atom spacer), Galα1-3Galβ1-4(3-deoxyGlcNAc)-HSA (#NGP2335), Galα1-3Galβ1-4GlcNAcβ1-HDPE (#NGL0334), Galα1-3Gal-BSA (#NGP0203, 3 atom spacer), Galα1-3Galβ1-3GlcNAc-BSA (#NGP0333, 3 atom spacer), Galα1-3Galβ1-3GlcNAc-HSA (#NGP2333, 3 atom spacer), Galal-3Galβ1-4(6-deoxyGlcNAc)-HSA (#NGP2336, 3 atom spacer), Galα1-3Galβ1-4Glc-HSA (#NGP2330, 3 atom spacer), Galα1-3Gal-BSA (#NGP1203, 14 atom spacer), Galα1-3Gal-HSA (#NGP2203, 3 atom spacer), Galα1-3Gal-HSA (#NGP3203, 14 atom spacer), Galα1-3Galβ1-4GlcNAc-BSA (#NGP0334, 3 atom spacer), Galα1-3Galβ1-4GlcNAc-BSA (#NGP1334, 14 atom spacer), Galα1-3Galβ1-4GlcNAc-HSA (#NGP2334, 3 atom spacer), Galα1-3Galβ1-4GlcNAc-HSA (#NGP3334, 14 atom spacer), and Galα1-3Galβ1-4Glc-BSA (#NGP0330, 3 atom spacer), Galα1-3Galβ1-HDPE (#NGL0203),
- galactose-α-1,3-galactose oligosaccharides, such as those commercially available from Elicityl, including without limitation Galα1-3Galβ1-3(Fucα1-4)GlcNAc (#GLY076), Galα1-3Galβ1-4(Fucα1-3)GlcNAc (#GLY075), Galα1-3[Galβ1-4GlcNAcβ1-3]4Galβ1-4Glc (#GLY079), Galα1-3[Galβ1-4GlcNAcβ1-3]3Galβ1-4Glc (#GLY078), Galα1-3Galβ1-4Glc (#GLY070), Galα1-3[Galβ1-4GlcNAcβ1-3]2Galβ1-4Glc (#GLY077), Galα1-3Galβ1-4GlcNAcβ1-3Galβ1-4Glc (#GLY071), Galα1-3Galβ1-4GlcNAc (#GLY74-2, linear B-2 trisaccharide), and Galα1-3Galβ1-3GlcNAc (#GLY74-1), and
- additional macromolecules with α-Gal epitopes that are suitable for injection and *subsequent in situ* binding to anti-Gal antibodies include, without limitation:
   o mouse laminin with 50-70 α-gal epitopes (Galili U 1993 Springer Seminars in Immunopathology 15, 155), commercially available from Life Technologies (#23017-015),
   o multiple synthetic α-gal epitopes linked to BSA (Stone KR et al. 2007 Transplantation 83(2): 211-219),
   o GAS914, commercially produced by Novartis (WO9847915) and disclosed in Zhong R et al. 2003 Transplantation 75(1): 10-19,
   o the α-Gal polyethylene glycol conjugate TPC (Schirmer JM et al. 2004 Xenotransplantation 11(5): 436-443), and
   o α-Gal epitope-mimicking peptides linked to a macromolecule backbone (Sandrin MS et al. 1997 Glycoconj J 14(1): 97-105), and peptides encoded by mucin genes MUC1, 3 and 4.

In a more preferred embodiment, the agent comprising a terminal α-galactosyl moiety according to the invention is GAS914.

Antibiotics of the composition of the invention include, without limitation, carbapenems, cephalosporins, monobactams, penicillins, polypeptides, quinolones, sulfonamides and tetracyclines.

In another aspect, the invention is related to a composition comprising
(i) an agent comprising a terminal α-galactosyl moiety, and
(ii) an antibiotic
for use in medicine.

In an alternative embodiment, the invention relates to the use of a composition comprising
(i) an agent comprising a terminal α-galactosyl moiety, and
(ii) an antibiotic
for the manufacture a medicament for the prevention and or treatment of infection caused by bacteria of the gastrointestinal tract.

In a still alternative embodiment, the invention relates to a method for the treatment of a disease that comprises administering to a subject in need thereof a therapeutically effective amount of a composition comprising
(i) an agent comprising a terminal α-galactosyl moiety, and
(ii) an antibiotic

In another aspect, the invention is related to a composition comprising
(i) an agent comprising a terminal α-galactosyl moiety, and
(ii) an antibiotic
for use in the prevention and or treatment of infection caused by bacteria of the gastrointestinal tract.

Alternatively, the invention relates to the use of a composition comprising
(i) an agent comprising a terminal α-galactosyl moiety, and
(ii) an antibiotic
for the manufacture of a medicament for the prevention and/or treatment of infection caused by bacteria of the gastrointestinal tract.

In a still alternative embodiment, the invention relates to a method for the prevention and/or treatment of inflammation in a subject that comprises administering to a subject in need thereof a therapeutically effective amount of a composition comprising
(i) an agent comprising a terminal α-galactosyl moiety, and
(ii) an antibiotic

Agents comprising a terminal α-galactosyl moiety in the composition for use according to the invention have been described previously. In a particular embodiment, the terminal α-galactosyl is selected from the group comprising terminal Galα1-3Gal, terminal Galα1-2Gal, terminal Galα1-6Gal, terminal Galα1-6Glc, and terminal α-galactose sugar unit(s) capable of binding an anti-Gal antibody. In a more particular embodiment, the terminal α-galactosyl is terminal Galα1-3Gal. In a preferred embodiment, the agent is selected from the following:
- galactose-α-1,3-galactose, oligomers (e.g., dimers, trimmers, tetramers, pentasaccharides) of galactose-α-1,3-galactose, and derivatives thereof, including glucosamine derivatives, linear B-2 trisaccharide (Galα1-3Ga1β1-4GlcNAc, CAS No. 101627-01-4), linear B-6 trisaccharide (Galα1-3Galβ1-4Glc, CAS No. 56038-36-9) and derivatives thereof, α1-3 galactobiosyl β-methyl glycoside; α1-3, β1-4 galactotriose (Galα1-3Galβ1-4Gal, CAS No. 56038-36-9), galactotetraose (Galα1-3Galβ1-4Galα1-3-D-Gal, CAS No. 56038-38-1), Galili pentasaccharide (L537, Gal-α1,3Gal-β1,4GlcNAc-β1,3Gal-β1,4Glc, CAS No. 119502-59-9), and derivatives of the above such as, e.g., amino acid derivatives (e.g., dodecalysine, glycine), amides, esters, ethers, amines, sulfonamides, thioethers, acetals, carbamates, ureas and amidines,
- glycopeptides comprising oligosaccharides with terminal galactose-α-1,3-galactose,
- commercially available glycolipids (Dextra Laboratories, Ltd., United Kingdom) such as Galα1-3Gal glycolipids comprising blood group B type 2 linear trisaccharide (GN334, Galα1-3Galβ1-4GlcNac, CAS No. 101627-01-4); and comprising Galili pentasaccharide (L537, Gal-α1,3Gal-β1,4GlcNAc-β1,3Gal-β1,4Glc, CAS No. 119502-59-9),
- commercially available glycoconjugates with galactose-α-1,3-galactose epitopes available from Dextra include for instance: Galα1-3Gaβ1-4Glc-BSA (#NGP0330, 3 atom spacer), Galα1-3Galβ1-4(3-deoxyGlcNAc)-HSA (#NGP2335), Galα1-3Galβ1-4GlcNAcβ1-HDPE (#NGL0334), Galα1-3Gal-BSA (#NGP0203, 3 atom spacer), Galα1-3Galβ1-3GlcNAc-BSA (#NGP0333, 3 atom spacer), Galα1-3Galβ1-3GlcNAc-HSA (#NGP2333, 3 atom spacer), Galal-3Galβ1-4(6-deoxyGlcNAc)-HSA (#NGP2336, 3 atom spacer), Galα1-3Galβ1-4Glc-HSA (#NGP2330, 3 atom spacer), Galα1-3Gal-BSA (#NGP1203, 14 atom spacer), Galα1-3Gal-HSA (#NGP2203, 3 atom spacer), Galα1-3Gal-HSA (#NGP3203, 14 atom spacer), Galα1-3Galβ1-4GlcNAc-BSA (#NGP0334, 3 atom spacer), Galα1-3Galβ1-4GlcNAc-BSA (#NGP1334, 14 atom spacer), Galα1-3Galβ1-4GlcNAc-HSA (#NGP2334, 3 atom spacer), Galα1-3Galβ1-4GlcNAc-HSA (#NGP3334, 14 atom spacer), and Galα1-3Galβ1-4Glc-BSA (#NGP0330, 3 atom spacer), Galα1-3Galβ1-HDPE (#NGL0203),
- galactose-α-1,3-galactose oligosaccharides, such as those commercially available from Elicityl, including without limitation Galα1-3Galβ1-3(Fucα1-4)GlcNAc (#GLY076), Galα1-3Galβ1-4(Fucα1-3)GlcNAc (#GLY075), Galα1-3[Galβ1-4GlcNAcβ1-3]4Galβ1-4Glc (#GLY079), Galα1-3[Galβ1-4GlcNAcβ1-3]3Galβ1-4Glc (#GLY078), Galα1-3Galβ1-4Glc (#GLY070), Galα1-3[Galβ1-4GlcNAcβ1-3]2Galβ1-4Glc (#GLY077), Galα1-3Galβ1-4GlcNAcβ1-3Galβ1-4Glc (#GLY071), Galα1-3Galβ1-4GlcNAc (#GLY74-2, linear B-2 trisaccharide), and Galα1-3Galβ1-3GlcNAc (#GLY74-1), and
- additional macromolecules with α-Gal epitopes that are suitable for injection and *subsequent in situ* binding to anti-Gal antibodies include, without limitation:
   o mouse laminin with 50-70 α-gal epitopes (Galili U 1993 Springer Seminars in Immunopathology 15, 155), commercially available from Life Technologies (#23017-015),
   o multiple synthetic α-gal epitopes linked to BSA (Stone KR et al. 2007 Transplantation 83(2): 211-219),
   o GAS914, commercially produced by Novartis (WO9847915) and disclosed in Zhong R et al. 2003 Transplantation 75(1): 10-19,
   o the α-Gal polyethylene glycol conjugate TPC (Schirmer JM et al. 2004 Xenotransplantation 11(5): 436-443), and
   o α-Gal epitope-mimicking peptides linked to a macromolecule backbone (Sandrin MS et al. 1997 Glycoconj J 14(1): 97-105), and peptides encoded by mucin genes MUC1, 3 and 4.

In a more preferred embodiment, the agent comprising a terminal α-galactosyl moiety for use according to the invention is GAS914.

Antibiotics in the composition for use according to the invention have been described previously.

In a particular embodiment, the infection is caused by bacteria of the gastrointestinal tract, wherein the bacteria of the gastrointestinal tract are enteric bacteria. More particularly, the enteric bacteria are selected from the group consisting of bacteria of the genus *Acinetobacter, Actinomyces, Bacteroides, Bifidobacterium, Campylobacter, Clostridium, Corynebacterium, Enterococcus, Eubacterium, Fusobacterium, Haemophilus, Helicobacter, Lactobacilus, Mobiluncus, Peptostreptococcus, Porphyromonas, Prevotella, Propionibacterium, Pseudomonas, Staphylococcus, Streptococcus,* and *Veillonella,* and the Enterobacteriaceae family. More particularly, the enteric bacteria of the Enterobacteriaceae family is selected from the group consisting of bacteria of the genus *Citrobacter, Enterobacter, Escherichia, Klebsiella, Proteus, Salmonella Serratia* and *Yersinia.*

In a particular embodiment, the infection occurs in blood, gastrointestinal tract, heart, cardiovascular system, liver, lung, respiratory tract, kidney, urinary tract, nervous central system, skin, subcutaneous tissues and surgical wounds.

In a particular embodiment, the subject has endogenous anti-Gal antibodies. In a preferred embodiment, the subject is a human.

The invention is described in detail below by means of the following examples which are to be construed as merely illustrative and not limitative of the scope of the invention.

### EXAMPLES

### Example 1. Removal of anti-Gal antibodies prevents sepsis in Gal knockout mice

A knockout mouse lacking the α-1,3-galactosyltransferase (α1,3GT) gene was used. This mouse generates natural anti-Gal antibodies without any need of additional immunization, thereby allowing the inventors to investigate whether the removal of anti-Gal antibodies with GAS914 as unique action might have an impact in the sepsis developed in Gal knockout mice after colon ligation and puncture (CLP). In this model a total of 0.5 cm of cecum was ligated and punctured twice using a 30-gauge needle to allow protrusion of contents of cecum and assure the presence of bacteria in the peritoneum.

To evaluate the clinical status of the mice, the physical activity was analyzed using an established welfare scoring system that evaluates spontaneous activity, food intake and response to exogenous stimuli. A total of 34 animals were included in the study. Seventeen (control group) did not receive any treatment. The other 17 (GAS914 group) were treated with 10 mg/kg of GAS914 intraperitoneally every other day from day -3 of CLP and thereafter. The dosage and interval of GAS914 injections were selected based on previous local studies that showed the complete removal of anti-Gal antibodies on day 0 (CLP day). Essentially, all anti-Gal antibodies are eliminated with the initial GAS914 injection.

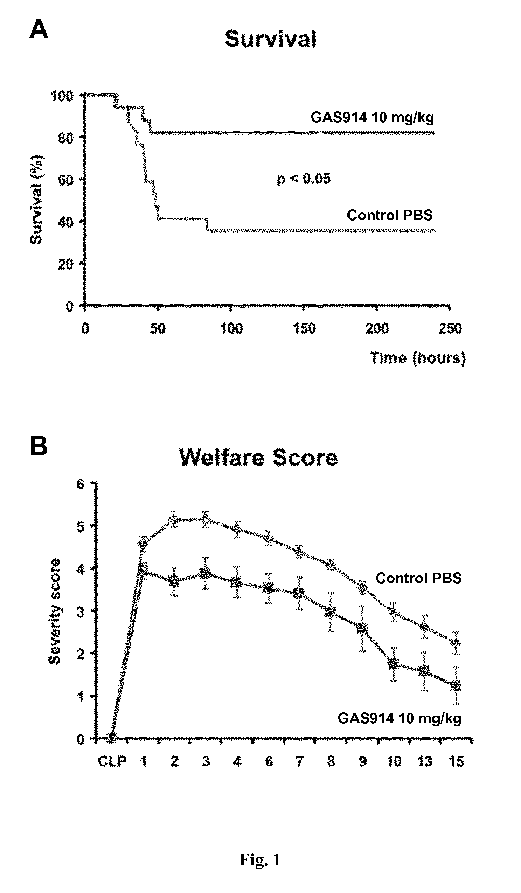

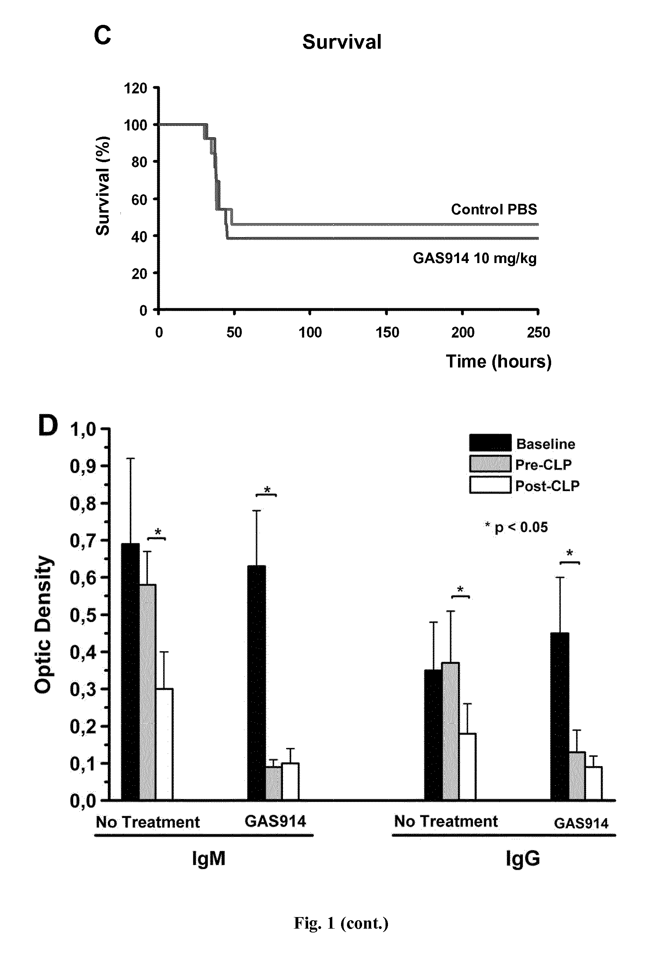

As it is shown in Figure 1A, 4 of the 17 (24%) Gal knockout mice treated with GAS914 died after CLP compared with 11 (65%) of untreated animals (p=0.01), with most of deaths occurring within 48h after CLP. Also, welfare of animals was better from day one after CLP in GAS914 treated animals compared to controls (Figure 1B). To obtain the benefit of removing anti-Gal antibodies in sepsis, these have to be depleted before CLP. The inventors also investigated in two groups of 10 animals, whether removal of anti-Gal antibodies with GAS 914, 12h after CLP had an impact in animal survival and welfare. In this case, GAS914 did not provide any benefit compared to control animals (Figure 1C).

Treatment with GAS914 before CLP was associated with nearly the depletion of all anti-Gal IgM and IgG antibodies at the time of CLP (Figure 1D). These antibodies barely change after CLP, whilst in non-treated animals there was a significant reduction of anti-Gal IgM and IgG antibodies after CLP, suggesting the presence of the antigen after the procedure (Figure 1D). Despite of this, no differences were observed 24 h after CLP between animals that did or did not receive GAS914 regarding the number of bacteria isolated in the blood, the type (*Escherichia coli* in all the cases) and the genotype of these microorganisms.

### Example 2. Removal of anti-Gal antibodies increases serum bactericidal activity

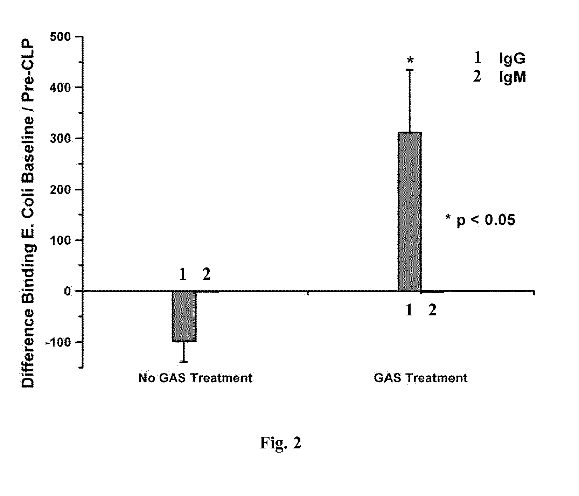

The authors of the invention observed that the binding of natural antibodies to bacterial blood isolates after CLP was modified by GAS914. Removal of anti-Gal antibodies was associated with an increase of reactivity of other IgG antibodies from gal-KO mice to *E. coli* (Figure 2). Therefore, CLP in Gal-KO mice caused a sepsis with blood isolation *of E. coli* that are bound by anti-Gal antibodies, and the removal of anti-Gal antibodies with GAS914 allowed the reaction of other IgG antibodies to the same E. *coli.*

*E. coli* isolated from Gal-KO mouse blood after CLP and *E. coli* O86:B7 (ATCC) were incubated overnight in Difco Nutrient Broth (Becton Dickinson) on a shaker at 37°C. The following day, the bacterial suspension was diluted 100 times using fresh medium. Sterile baby rabbit complement (Serotec) was added to the bacterial suspension (30 µl/mL). The mix was then incubated with 50 µl/mL of heat inactivated mouse serum samples (with and without anti-Gal antibodies), during 12 hours at 37°C. Serum bactericidal capacity was assessed by plating hourly an adequate dilution of the culture on 1.5 % (w/v) agar medium plates for 18 h at 37°C. The following day bacterial colonies were counted. Broth alone was used as a negative control for bacterial growth and complement without added mouse serum was used as a positive control for killing via the alternative complement pathway. *E. coli* O86:B7 (ATCC).

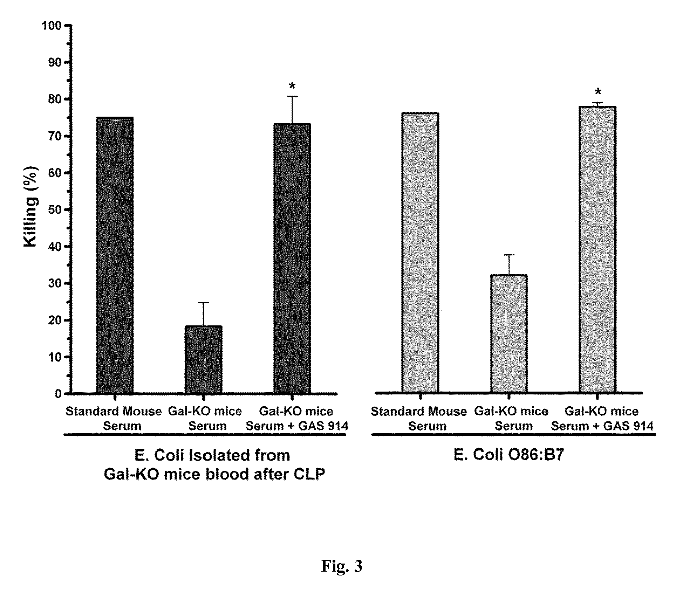

The augment of binding of antibodies to blood isolates of *E. coli* led to an increase in direct serum bactericidal activity of Gal-KO mice serum (Figure 3). A commercial mice serum pool (Sigma S3509) showed 75% and 74% on the complement-mediated antibody killing *of E. coli* isolated from Gal-KO mice after CLP and *E. coli* 086:B7 strain (Figure 3). The latter was used as control of bacterium that effectively binds alpha-Gal antibodies (Posekany KJ et al. 2002 Infect Immun 70 (11):6215-6222; Lamontagne A et al. 2013 PLoS One 8(6):e64992). Gal-KO serum mice carrying anti-Gal antibodies evidenced an average killing of 18.3% and 32%, respectively, against the two *E. coli,* which increased to 73% and 78%, respectively, after the depletion of anti-Gal antibodies with GAS914 (p<0.05 for both augments).

All together, these data substantiate that GAS914 in Gal-KO mice increased the serum antibody IgG reactivity and bactericidal activity to blood *E. coli* isolates, to levels similar as those observed in wild-type mice lacking anti-Gal antibodies.

### Example 3. Serum anti-carbohydrate pattern after removal of anti-Gal antibodies by GAS914

The pattern of serum anti-carbohydrate antibodies before and after treatment with GAS914 was investigated in a glycan array containing 435 glycan antigens and 141 bacterial antigens. The only carbohydrate antigens that showed reductions after GAS914 treatment were Gal trisaccharide, Gal tetrasaccharide and Gal pentasaccharide, with levels that dropped 96.5%, 95.%% and 90%, respectively, compared to those before treatment. In addition, there were also statistically significant augments against 48 carbohydrate and 9 bacterial antigens. Many of the augments corresponded to relative fluorescence units (RFU) below 1000. However, these increases included two structures of Fuca1-2Galb1-3(Fuca1-4)GlcNAcb that is expressed in *E. coli* O86:B7, one of the bacteria in which it was observed an increase in bactericidal activity with the removal of anti-Gal antibodies. Therefore, it is possible that statistically significant changes are relevant occurring even with values below 1000. Carbohydrates and bacterial antigens showing statistically significant increases after depletion of anti-Gal antibodies with values beyond 1500 RFU included the following:
1. Chitotriose, chitopentaose and chitohexaose, which showed statistically significant increases with high values. These GlcNAc residues were also expressed in two bacterial antigens also showing statistically significant changes with high values: *Salmonella enterica* 062 and *Pseudomonas aeruginosa* 03.
2. Another carbohydrate with significant augments and high values was hyaluronic acid with 8 disaccharides. Hyaluronic acid is expressed on *E. coli* 0161 that is the bacterial antigen with the highest statistically significant change after GAS914 treatment.
3. Galb1-4GalNAcb residues, b-mannose and sialyl-lea also had statistically significant changes and high values.
4. *Salmonella enterica* 047 showed statistically significant changes with high values.

## Claims

1. An agent comprising a terminal α-galactosyl moiety, for use in the prevention and/or treatment of an infection in a subject, wherein said infection is caused by bacteria of the gastrointestinal tract.

2. The agent for use according to the preceding claim, wherein the bacteria of the gastrointestinal tract are enteric bacteria.

3. The agent for use according to the preceding claim, wherein the enteric bacteria are selected from the group consisting of bacteria of the genus *Acinetobacter, Actinomyces, Bacteroides, Bifidobacterium, Campylobacter, Clostridium, Corynebacterium, Enterococcus, Eubacterium, Fusobacterium, Haemophilus, Helicobacter, Lactobacilus, Mobiluncus, Peptostreptococcus, Porphyromonas, Prevotella, Propionibacterium, Pseudomonas, Staphylococcus, Streptococcus* and *Veillonella,* and bacteria of the Enterobacteriaceae family.

4. The agent for use according to the preceding claim, wherein the enteric bacteria of the Enterobacteriaceae family are selected from the group consisting of bacteria of the genus *Citrobacter, Enterobacter, Escherichia, Klebsiella, Proteus, Salmonella, Serratia,* and *Yersinia.*

5. The agent for use according to any of the preceding claim, wherein the infection occurs in blood, gastrointestinal tract, heart, cardiovascular system, liver, lung, respiratory tract, kidney, urinary tract, nervous central system, skin, subcutaneous tissues and surgical wounds.

6. The agent for use according to any of the preceding claims, wherein the terminal α-galactosyl moiety is comprised within a group selected from the group consisting of a terminal Galα1-3Gal, a terminal Galα1-2Gal, a terminal Galα1-6Gal, a terminal Galα1-6Glc, and a terminal α-galactose sugar unit(s) capable of binding an anti-Gal antibody.

7. The agent for use according to any of the preceding claims, wherein the terminal α-galactosyl is terminal Galα1-3Gal.

8. The agent for use according to the any of the preceding claims, wherein the agent is selected from the group comprising:
- galactose-α-1,3-galactose (Galα-3Gal),
- galactose-α-1,3-galactose oligomers and oligosacharides, comprising linear B-2 trisaccharide (Galα1-3Galβ1-4GlcNAc, CAS No. 101627-01-4), linear B-6 trisaccharide (Galα1-3Galβ1-4Glc, CAS No. 56038-36-9), α1-3 galactobiosyl β-methyl glycoside; α1-3, β1-4 galactotriose (Galα1-3Galβ1-4Gal, CAS No. 56038-36-9), galactotetraose (Galα1-3Galβ1-4Galα1-3-D-Gal, CAS No. 56038-38-1), Galili pentasaccharide (L537, Gal-α1,3Gal-β1,4GlcNAc-β1,3Gal-β1,4Glc, CAS No. 119502-59-9), Galα1-3Galβ1-3(Fucα1-4)GlcNAc (#GLY076), Galα1-3Galβ1-4(Fucα1-3)GlcNAc (#GLY075), Galα1-3[Galβ1-4GlcNAcβ1-3]4Galβ1-4Glc (#GLY079), Galα1-3[Galβ1-4GlcNAcβ1-3]3Galβ1-4Glc (#GLY078), Galα1-3Galβ1-4Glc (#GLY070), Galα1-3[Galβ1-4GlcNAcβ1-3]2Galβ1-4Glc (#GLY077), Galα1-3Galβ1-4GlcNAcβ1-3Galβ1-4Glc (#GLY071), Galα1-3Galβ1-4GlcNAc, and Galα1-3Galβ1-3GlcNAc (#GLY74-1), and derivatives thereof,
- galactose-α-1,3-galactose derivatives, comprising glucosamine derivatives, amides, esters, ethers, amines, sulfonamides, thioethers, acetals, carbamates, ureas and amidines,
- glycopeptides and glycoproteins comprising oligosaccharides with terminal galactose-α-1,3-galactose,
- glycolipids comprising galactose-α-1,3-galactose, comprising glycolipids comprising blood group B type 2 linear trisaccharide (GN334, Galα1-3Galβ1-4GlcNac, CAS No. 101627-01-4) and glycolipids comprising Galili pentasaccharide (L537, Gal-α1,3Gal-β1,4GlcNAc-β1,3Gal-β1,4Glc, CAS No. 119502-59-9),
- galactose-α-1,3-galactose glycoconjugates, comprising Galα1-3Galβ1-4Glc-BSA (#NGP0330, 3 atom spacer), Galα1-3Galβ1-4(3-deoxyGlcNAc)-HSA (#NGP2335), Galα1-3Galβ1-4GlcNAcβ1-HDPE (#NGL0334), Galα1-3Gal-BSA (#NGP0203, 3 atom spacer), Galα1-3Galβ1-3GlcNAc-BSA (#NGP0333, 3 atom spacer), Galα1-3Galβ1-3GlcNAc-HSA (#NGP2333, 3 atom spacer), Galal-3Galβ1-4(6-deoxyGlcNAc)-HSA (#NGP2336, 3 atom spacer), Galα1-3Galβ1-4Glc-HAS (#NGP2330, 3 atom spacer), Galα1-3Gal-BSA (#NGP1203, 14 atom spacer), Galα1-3Gal-HSA (#NGP2203, 3 atom spacer), Galα1-3Gal-HSA (#NGP3203, 14 atom spacer), Galα1-3Galβ1-4GlcNAc-BSA (#NGP0334, 3 atom spacer), Galα1-3Galβ1-4GlcNAc-BSA (#NGP1334, 14 atom spacer), Galα1-3Galβ1-4GlcNAc-HSA (#NGP2334, 3 atom spacer), Galα1-3Galβ1-4GlcNAc-HSA (#NGP3334, 14 atom spacer), and Galα1-3Galβ1-4Glc-BSA (#NGP0330, 3 atom spacer), Galα1-3Galβ1-HDPE (#NGL0203), and
- macromolecules comprising galactose-α-1,3-galactose, comprising mouse laminin, synthetic α-gal epitopes linked to BSA, GAS914, the α-Gal polyethylene glycol conjugate TPC, and α-Gal epitope-mimicking peptides linked to a macromolecule backbone.

9. The agent for use according to any of the preceding claims, wherein the agent is a the random copolymer GAS914 having the following structure: 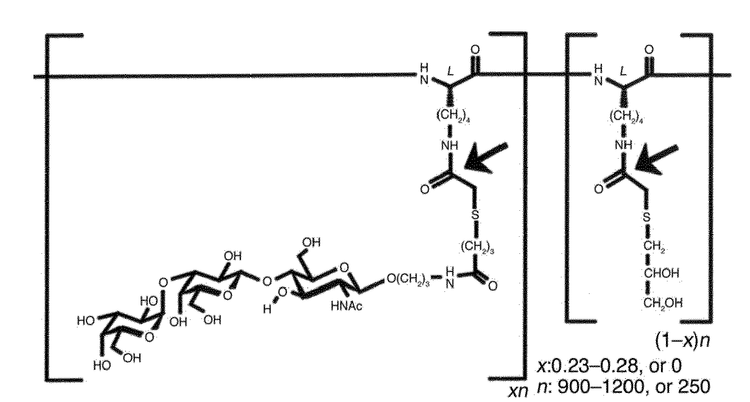 wherein n represents the average degree of polymerization, x represents the fraction of glycosylated monomer; and 1 - x represents the fraction of thioglycerol-capped monomer.

10. The agent for use according to any of the preceding claims, wherein the subject has endogenous anti-Gal antibodies.

11. The agent for use according to any of the preceding claims, wherein the subject is a human.

12. A composition comprising:
(i) an agent comprising a terminal α-galactosyl moiety, and
(ii) an antibiotic.

13. A composition according to the preceding claim, wherein the agent comprising a terminal α-galactosyl moiety is comprised within a group selected from the group consisting of a terminal Galα1-3Gal, a terminal Galα1-2Gal, a terminal Galα1-6Gal, a terminal Galα1-6Glc, and a terminal α-galactose sugar unit(s) capable of binding an anti-Gal antibody.

14. A composition according to any of claims 12 or 13 for use in medicine.

15. A composition according to any of claims 12 or 13 for use in the prevention and/or treatment of infection caused by bacteria of the gastrointestinal tract in a subject.
